# EUROPÄISCHE PATENTANMELDUNG

(11) **EP 2 255 810 A1**
(43) Veröffentlichungstag der Anmeldung: **01.12.2010**
(21) Anmeldenummer: 10177166.5
(22) Anmeldetag: 22.02.2006
(51) Int. Cl.: A61K 31/53, A61K 9/22, A61K 9/26, A61K 9/52

(54) **Arzneiformen mit kontrollierter Bioverfügbarkeit, die Vardenafil enthalten**

(30) Priorität: 01.03.2005 DE 102005009241
(62) Teilanmeldung aus: 06707140.7
(71) Anmelder: Bayer Schering Pharma AG, 13353 Berlin (DE)
(72) Erfinder: Serno, Peter Dr., 51467, Bergisch Gladbach (DE); Heinig, Roland Dr., 42115, Wuppertal (DE); Pauli, Kerstin Dr., 10585, Berlin (DE)

(57) **Zusammenfassung**

Die vorliegende Anmeldung betrifft neue Arzneimittelformulierungen von Vardenafil, die sich schnell im Mund auflösen und über eine kontrollierte Bioverfügbarkeit verfügen sowie Verfahren zu deren Herstellung.

## Beschreibung

Die vorliegende Anmeldung betrifft neue Arzneimittelformulierungen von Vardenafil, die sich schnell im Mund auflösen und über eine kontrollierte Bioverfügbarkeit verfügen sowie Verfahren zu deren Herstellung.

Imidazotriazinon-Derivate, wie Vardenafil sowie dessen Verwendung als cGMP Phosphodiesterase-Inhibitor und dessen Wirkungsspektrum sind bekannt (z.B. WO 99/24433) und unter dem Namen Levitra® im Markt erhältlich. Vardenafilhydrochlorid kann oral verabreicht werden, wobei verschiedene orale Darreichungsformen verwendet werden können wie beispielsweise Tabletten, Hartgelatinekapseln, Weichgelatinekapseln, Pulver, Granulate, Kautabletten oder Brausetabletten. Eine weitere Verabreichungs-Möglichkeit sind schnell im Mund zerfallende Darreichungsformen. Diese können vom Patienten zügig, diskret und ohne Flüssigkeit eingenommen werden. Diese Arzneiformen zerfallen in der Regel in weniger als 3 Minuten, vorzugsweise weniger als 1 Minute im Mund wobei die dabei entstehende Lösung oder Suspension dann geschluckt wird. Im Mund schnell zerfallende Darreichungsformen sind daher ganz besonders für Patienten geeignet, die Probleme beim Schlucken von Tabletten haben.

Techniken zur Herstellung von Darreichungsformen, die im Mund rasch zerfallen, sind allgemein bekannt. Beispielsweise durch Gefriertrocknung hergestellte Plättchen, wie in WO 93/23017 beschrieben; das Verpressen pulverförmiger Mischungen zu schnell zerfallenden Tabletten, wie in WO 03/051338 beschrieben; das Einarbeiten der Wirkstoff in Filme, wie in WO 00/42992 beschrieben.

Ein Problem bei der Formulierung von schnell im Mund zerfallenden Darreichungsformen stellt oft der bittere oder anderweitig inakzeptable Geschmack des Wirkstoffes dar. In diesen Fällen kann z.B. durch Überziehen des Wirkstoffes, von Wirkstoffgranulaten oder beschichteten Wirkstoffpellets mit Speichel- unlöslichen aber Magensaft-löslichen Polymeren ein Auflösen des Wirkstoffes im Mund im Anschluss an den Tablettenzerfall vollständig vermieden werden. Dieses Problem liegt bei Vardenafil und seinen Salzen, wie dem Vardenafil Hydrochlorid Trihydrat, nicht vor. Der Geschmack des Wirkstoffes ist nur leicht bitter und kann durch Zugabe üblicher Aromastoffe leicht überdeckt oder in eine angenehme Geschmacksempfindung eingebunden werden.

Es wurde jetzt jedoch festgestellt, dass bei derartigen nach bekannten Verfahren mit dem Wirkstoff Vardenafil Hydrochlorid Trihydrat hergestellten Darreichungsformen andere Probleme auftreten. Nach Verabreichung derartiger Darreichungsformen werden im Menschen Plasmakonzentrationsverläufe beobachtet, die von denen nach Gabe einer handelsüblichen Vardenafil Hydrochlorid Trihydrat - Tablette (Levitra^{®}) abweichen. Insbesondere kommt es zu höheren maximalen Plasmakonzentrationen und die biologische Verfügbarkeit des Wirkstoffes ist höher als nach Gabe der handelsüblichen Tablette. Dies kann bei Patienten, die bereits längere Zeit mit handelsüblichen Vardenafil Hydrochlorid Trihydrat - Schlucktabletten behandelt wurden und auf schnell im Mund zerfallende Tabletten umgestellt werden oder bei Patienten, die häufig in Abhängigkeit von ihrer Lebenssituation zwischen Schlucktabletten und schnell im Mund zerfallenden Tabletten hin und her wechseln, unerwünscht sein.

Es wurden überraschenderweise im Mund schnell zerfallende Formulierungen von Vardenafil gefunden, die diese unerwarteten und unerwünschten Veränderungen des pharmakokinetischen Profiles vermeiden. Mit diesen erfindungsgemäßen Formulierungen kann der Patient die Vorteile von schnell im Mund zerfallenden Arzneiformen wie die leichte Schluckbarkeit oder Einnehmbarkeit ohne Flüssigkeit ohne die beschriebenen Nachteile nutzen.

Entsprechend der vorliegenden Erfindung ist es hierzu notwendig, dass die Freisetzungsrate von Vardenafil nach der Applikation der schnell im Mund zerfallenden Darreichungsform limitiert wird. Es wurde ferner gefunden, dass sich diese Limitierung der Wirkstofffreisetzung durch die Messung der Lösegeschwindigkeit von Vardenafil in der USP Blattrührerapparatur in physiologischer Kochsalzlösung bei 37°C und 50 Umdrehungen pro Minute ermitteln lässt und mit dieser Bestimmungsmethode für die erfindungsgemäße Darreichungsform eine Freisetzungsrate festgelegt werden kann, wonach sich innerhalb der ersten 5 Minuten nicht mehr als 50 % der Dosis auflösen darf.

Zur Herstellung von Formulierungen, die dem erfindungsgemäßen Lösegeschwindigkeitskriterium genügen, wurden eine Reihe verschiedener Verfahren gefunden, die im Folgenden beschrieben werden.

Eine Möglichkeit zur Herstellung der erfindungsgemäßen Arzneimittelformulierung ist, Vardenafil in Form des Vardenafil Dihydrates oder des wasserfreien Vardenafils einzusetzen.

Das Vardenafil Dihydrat oder wasserfreies Vardenafil wird gemäß einem der bekannten Verfahren zur Herstellung von Arzneiformen, die im Mund schnell zerfallen, als Wirkstoff verarbeitet. Unter schnell im Mund zerfallender Arzneiform wird hierbei verstanden, dass die Zerfallszeit der Arzneiform (Methode der Europäischen Pharmakopoe) kürzer als 3 Minuten, vorzugsweise kürzer als 1 Minute ist.

Diese Arzneiformen werden durch Mischen des Wirkstoffes mit Zuckern, Zuckeralkoholen, Sprengmitteln oder anderen Zerfallsförderern sowie weiteren Hilfsstoffen wie Tenside, Schmiermittel, Fließregulierungsmittel Geschmackstoffe, Farbstoffe oder Füllstoffe und Verpressen auf einer Tablettenmaschine hergestellt. Alternativ kann das wasserfreie Vardenafil oder Vardenafil Dihydrat zusammen mit Hilfsstoffen wie Zuckeralkoholen, Polymeren oder Tensiden in einem wässrigen Lösungsmittel gelöst oder suspendiert werden, die Lösung oder Suspension wird in Blisternäpfe dosiert und einem Gefriertrocknungsprozess unterworden. Ebenfalls alternativ kann das wasserfreie Vardenafil oder Vardenafil Dihydrat zusammen mit Hilfsstoffen wie Filmbildnern, Weichmachern, Geschmacks- und Farbstoffen in einem organischen Lösungsmittel gelöst oder suspendiert werden und zu einem Film verarbeitet werden. Auch eine lösungsmittelfreie Filmherstellung mit schmelzbaren Filmformulierungen ist möglich. Nach der Herstellung werden die Filme auf die einer Einzeldosis entsprechenden Stücke geschnitten.

Eine weitere Möglichkeit zur Erzielung der erfindungsgemäß geringen Lösegeschwindigkeit von Vardenafil in physiologischer Kochsalzlösung ist die Verwendung eines Salzes von Vardenafil mit geringer Wasserlöslichkeit. Die Löslichkeit dieser Salze und somit auch Lösegeschwindigkeit kann durch Zugabe eines gleichionigen Zusatzes gegebenenfalls noch weiter zurückgedrängt werden.

Eine weitere Möglichkeit zur Erzielung der erfindungsgemäß geringen Lösegeschwindigkeit von Vardenafil in physiologischer Kochsalzlösung ist, ein wasserlösliches Vardenafil-Salz vorher derart zu behandeln, so dass die erfindungsgemäße Freisetzungsrate erzielt wird. Hierzu eignen sich insbesondere das Überziehen der Wirkstoff-Salze mit oder das Einbetten in Polymere(n). Die Vardenafil-Salze können dabei lösungsmittelfrei oder lösungsmittelhaltig sein und in einer unterschiedlichen polymorphen Form vorliegen. Beispiele für wasserlösliche Salze sind Vardenafil Hydrochlorid Trihydrat, Vardenafil Dimesilat Monohydrat oder Vardenafil Monomesilat. Es sind aber auch Salze des Vardenafil mit Citronensäure, Weinsäure, Bernsteinsäure, Schwefelsäure, Essigsäure, Adipinsäure, Gluconsäure, Glucuronsäure, Glutaminsäure, Glutarsäure, Glycerophosphorsäure, Milchsäure, Maleinsäure, Äpfelsäure, Phosphorsäure, Lactobionsäure, Malonsäure, Naphtalenesulfonsäure, Naphtalenedisulfonsäure oder Toluolsulfonsäure möglich. Alternativ ist es auch möglich, das oder die wasserlöslichen Vardenafil-Salz(e) durch gemeinsames Verarbeiten von Vardenafil und Säure in der Arzneiform zu erhalten. In diesem Fall bildet sich das entsprechende Salz nach Zutritt von wässrigem Medium im Mund. Die erfindungsgemäße Freisetzungsgeschwindigkeit durch Überziehen oder Einbetten mit Polymeren kann pH- oder zeitkontrolliert erreicht werden. Zur pH - kontrollierten Freisetzung eignen sich physiologisch verträgliche Polymere, die bei neutralem pH unlöslich sind und bei saurem pH löslich sind, insbesondere basisches Butyl methacrylat Copolymer (z.B. Eudragit^{®} E 100). Die zeitkontrollierte Freisetzung wird durch Überziehen oder Einbetten mit physiologisch verträglichen Polymeren, beispielhaft und vorzugsweise mit Ethylcellulose, erreicht. Diese limitieren zunächst die Wirkstofffreisetzung auf die erfindungsgemäße Rate, geben dann aber nachfolgend den Wirkstoff durch Diffusion oder Aufreißen des Filmes frei.

Zum Überziehen des Wirkstoffes mit Polymer werden Wirkstoffkristalle, -granulat oder -pellets, in einer geeigneten Apparatur und nach geeigneten Verfahren, wie in der Wirbelschicht oder einem Trommelcoater, mit Polymerlösung oder -schmelze überzogen. Auch ein Überziehen in Sprühtrocknungs- oder Sprüherstarrungsverfahren sind möglich. Ein Überziehen des Wirkstoffes kann auch in einem Coazervationsverfahren erreicht werden. Zur Einbettung des Wirkstoffes wird dieser gemeinsam mit Polymer auf einer Walze oder Tablettenmaschine verpresst. Auch das gemeinsame Ausfällen von Wirkstoff und Polymer in einem Copräcipitatverfahren sind möglich.

Zur Verarbeitung des bevorzugt verwendeten Polymers Poly(butylmethacrylat-co-(2-dimethylaminoethyl)methacrylat-co-methylmethacrylat) (Eudragit^{®} E 100) wird das Polymer entweder in Aceton/Isopropanol/Wasser gelöst oder es wird eine wässrige Dispersion der fein vermahlenen Substanz hergestellt, die neben Polymer und Wasser auch Tenside wie Natriumlaurylsulfat und Trennmittel wie Magnesiumstearat enthält. Die so erhaltene Lösung oder Dispersion wird auf den Wirkstoff enthaltende Partikel, beispielsweise ein Granulat, aufgesprüht. Hierzu eignen sich Wirbelschichtverfahren, beispielsweise eine Lackierung im Wurster - Rohr oder das Aufsprühen in Coatern. Eine typische Auftragsmenge beträgt dabei beispielsweise 1 mg Polymer pro cm² Oberfläche der Partikel.

Eine weitere Möglichkeit zur Erzielung der erfindungsgemäß geringen Auflösungsgeschwindigkeit in physiologischer Kochsalzlösung ist, die Verwendung grober Partikelgrößenfraktionen eines Vardenafil - Salzes, beispielsweise von Vardenafil Hydrochlorid Trihydrat, insbesondere von Vardenafil Hydrochlorid Trihydrat mit einer mittleren Partikelgröße > 80 µm. Zur weiteren Reduktion der Auflösungsgeschwindigkeit können die Partikel auch überzogen werden.

Nachdem das Vardenafil-Salz in einem der beschriebenen Verfahren so vorbehandelt wurde, dass die erfindungsgemäße Freisetzungsgeschwindigkeit erzielt werden kann, wird es gemäß einem der bekannten Verfahren zur Herstellung von Arzneiformen, die im Mund schnell zerfallen, als Wirkstoff verarbeitet.

Hierzu eignen sich das Mischen des vorbehandelten Wirkstoffes mit Zuckern, Zuckeralkoholen, Sprengmitteln oder anderen Zerfallsförderern sowie weiteren Hilfsstoffen wie Tenside, Schmiermittel, Fließregulierungsmittel Geschmackstoffe, Farbstoffe oder Füllstoffe und Verpressen auf einer Tablettenmaschine. Alternativ kann das vorbehandelte Vardenafil - Salz zusammen mit Hilfsstoffen wie Zuckeralkoholen, Polymeren oder Tensiden in einem wässrigen Lösungsmittel suspendiert werden, die Suspension wird in Blisternäpfe dosiert und einem Gefriertrocknungsprozess unterworden. Ebenfalls alternativ kann das vorbehandelte Vardenafil - Salz zusammen mit Hilfsstoffen wie Filmbildnern, Weichmachern, Geschmacks- und Farbstoffen in einem organischen Lösungsmittel suspendiert werden und zu einem Film verarbeitet werden. Auch eine lösungsmittelfreie Filmherstellung mit schmelzbaren Filmformulierungen ist möglich. Nach der Herstellung werden die Filme auf die einer Einzeldosis entsprechenden Stücke geschnitten.

### Vergleichsbeispiel 1: Übermäßige Bioverfügbarkeit und erhöhte maximale Plasmakonzentration nach Gabe nicht erfindungsgemäßer, schnell im Mund zerfallender Tabletten

Durch Mischen aller Bestandteile und Direktverpressen auf einer Rundläufer - Tablettenmaschine werden Tabletten hergestellt, die aus 23.7 mg Vardenafil hydrochlorid trihydrat, 0,748 mg gelbem Eisenoxid, 0,102 mg rotem Eisenoxid, 1,02 mg Aprikosenaroma, 0,17 mg Neohesperidin-Dihydrochalcone, 3,40 mg Aspartam 0,850 mg hochdispersem Siliciumdioxid, 4,25 mg Magnesiumstearat und 135,76 mg Pharmaburst^{®} (handelsübliche Mischung der Firma SPI) bestehen. Die Wirkstofffreisetzung in 900 ml physiologischer Kochsalzlösung bei 37°C und 50 Umdrehungen pro Minute in der USP Blattrührerapparatur beträgt 85 % in 5 Minuten. Somit ist das erfindungsgemäße Lösegeschwindigkeitskriterium nicht erfüllt. Als Vergleich wird im Cross-over - Verfahren an 12 Probanden eine mit Wasser zu schluckende Tablette gegeben, die aus folgenden Bestandteilen besteht: 23.705 mg Vardenafil Hydrochlorid Trihydrat (entsprechend 20 mg Vardenafil), 141,797 mg Mikrokristalline Cellulose, 8,85 mg Quervernetztes Polyvinylpyrrolidon, 0,885 mg kolloidales Siliciumdioxid, 1,770 mg Magnesiumstearat, 3,385 mg Hypromellose, 1,128 mg Macrogol 400, 0,925 mg Titandioxid, 0,188 mg gelbes Eisenoxid und 0,015 mg rotes Eisenoxid. Nach Applikation der handelsüblichen Schlucktablette (Levitra^{®}) ergibt sich eine maximale Plasmakonzentration von 20.1 µg/l (geometrisches Mittel), nach Applikation der nicht erfindungsgemäßen, schnell im Mund zerfallenden Tablette dieses Vergleichsbeispieles eine maximale Plasmakonzentration von 25.1 µg/l (geometrisches Mittel). Die relative Bioverfügbarkeit der nicht erfindungsgemäßen, im Mund zerfallenden Tablette beträgt 128 %.

### Beispiel 2: Nachweis annähernd übereinstimmender Bioverfügbarkeit einer erfindungsgemäßen, schnell im Mund zerfallenden Tablette mit einer Schlucktablette

Eine im Mund zerfallende Tablette bestehend aus 10.7 mg Vardenafildihydrat (entsprechend 10 mg Vardenafil), 0,484 mg gelbem Eisenoxid, 0,066 mg rotem Eisenoxid, 1,1 mg Aprikosenaroma, 4,4 mg Aspartam, 6,6 mg Magnesiumstearat und 196,65 mg Pharmaburst^{®} B2 (handelsübliche Hilfsstoffmischung der Firma SPI) wird hergestellt indem Vardenafildihydrat, gelbes Eisenoxid, rotes Eisenoxid, Aprikosenaroma, Aspartam, und Pharmaburst^{®} in einem Zwangsmischer gemischt werden und diese Mischung mit Magnesiumstearat in einem Freifallmischer nachgemischt wird. Diese schnell im Mund zerfallende Tablette ist erfindungsgemäß, da sich in physiologischer Kochsalzlösung bei 37 °C in 5 Minuten weniger als 9 % der Dosis löst und somit das erfindungsgemäße Lösegeschwindigkeitskriterium erfüllt wird. Im Cross-over - Vergleich an 11 Probanden wird die relative Bioverfügbarkeit im Vergleich zu einer Referenztablette folgender Zusammensetzung untersucht: 11,852 mg Vardenafil Hydrochlorid Trihydrat (entsprechend 10 mg Vardenafil), 105,023 mg Mikrokristalline Cellulose, 6,25 mg Quervernetztes Polyvinylpyrrolidon, 0,625 mg kolloidales Siliciumdioxid, 1,25 mg Magnesiumstearat, 2,391 mg Hypromellose, 0,797 mg Macrogol 400, 0,653 mg Titandioxid, 0,133 mg gelbes Eisenoxid und 0,011 mg rotes Eisenoxid. Als Maß für die biologische Verfügbarkeit der Prüfformulierungen wurde die Fläche unter der Plasma - Konzentrations - Zeitkurve (AUC) herangezogen, welche für die erfindungsgemäße Tablette 34,9 µg*h/L und für die Referenztablette 35,7 µg*h/L (jeweils geometrische Mittel) betrugen. Die maximale Plasmakonzentration der erfindungsgemäßen Tablette konnte auf 79 % der Referenztablette begrenzt werden.

### Beispiel 3: Nachweis annähernd übereinstimmender Bioverfügbarkeit einer erfindungsgemäßen, schnell im Mund zerfallenden Tablette mit einer Schlucktablette

11 Probanden erhalten jeweils eine im Mund zerfallende Tablette bestehend aus 10.7 mg Vardenafildihydrat (entsprechend 10 mg Vardenafil), 5 mg gemahlener Bernsteinsäure, 0,484 mg gelbem Eisenoxid, 0,066 mg rotem Eisenoxid, 1,1 mg Aprikosenaroma, 4,4 mg Aspartam, 6,6 mg Magnesiumstearat und 191,65 mg Pharmaburst^{®} B2 (handelsübliche Hilfsstoffmischung der Firma SPI). Diese schnell im Mund zerfallende Tablette ist erfindungsgemäß, da die Wirkstofffreisetzung in 900 ml physiologischer Kochsalzlösung bei 37 °C und 50 Umdrehungen pro Minute in der USP Blattrührerapparatur nur 40 % in 5 Minuten beträgt und somit das erfindungsgemäße Lösungsgeschwindigkeitskriterium erfüllt wird. Im Cross-over - Vergleich zu der im Vergleichsbeispiel 2 aufgeführten Referenztablette beträgt die relative Bioverfügbarkeit 101,8 %.

### Beispiel 4: Nachweis annähernd übereinstimmender Bioverfügbarkeit einer erfindungsgemäßen, schnell im Mund zerfallenden Tablette mit einer Schlucktablette

118 g wasserfreies Vardenafil, 590 g Mannit und 11,8 g Poloxamer 188 werden in 2360 g Wasser suspendiert, bzw. gelöst. Die Suspension wird in einer Wirbelschichtapparatur auf 848 g Pharmaburst^{®} B2 (handelsübliche Hilfsstoffmischung der Firma SPI) und 44.8 g Aspartam aufgesprüht. Das Granulat wird in der Wirbelschicht getrocknet und mit 2714 g Pharmaburst^{®} B2, 22,42 g pulverförmigem Orangenaroma und 134,5 g Magnesiumstearat nachgemischt. Diese Mischung wird auf einer Rundlauftablettenpresse zu runden Tabletten von 11 mm Durchmesser und 380 mg Masse verpresst. Die Freisetzungsrate dieser schnell im Mund zerfallende Tablette beträgt 49 % in 5 Minuten bei 37°C. In einem Cross-over - Vergleich an 11 gesunden Probanden wird die Pharmakokinetik dieser im Mund zerfallenden Tablette gegen die in Beispiel 2 beschriebene Referenztablette getestet. Es ergibt sich eine mittlere relative Bioverfügbarkeit von 92 % und eine mittlere maximale Plasmakonzentration von 84 % der Referenztablette.

### Beispiel 5

Poly(butylmethacrylat-co-(2-dimethylaminoethyl)methacrylat-co-methylmethacrylat) (Eudragit^{®} E 100) wird auf einer Fließbett - Gegenstrahlmühle vermahlen. 152,07 g des vermahlenen Produktes wird mit 47,93 g mikronisiertem Vardenafil Hydrochlorid Trihydrat gemischt, gesiebt und nochmals gemischt. Die Mischung wird auf einer Walze kompaktiert und über ein 1 mm Sieb zerkleinert. 14,31 g des so erhaltenen Granulates werden mit 0,55 g pulverförmigem Orangenaroma, 1,1 g Aspartam, 90,74 g Pharmaburst^{®} B2 und 3.3 g Magnesiumstearat gemischt und zu Tabletten von 11 mm Durchmesser und einer Masse von 380 mg verpresst. Die so erhaltenen, schnell im Mund zerfallenden Tabletten setzen in der USP Blattrührerapparatur in 900 ml physiologischer Kochsalzlösung bei 37°C und 50 Umdrehungen pro Minute 42 % des Wirkstoffes in 5 Minuten frei.

### Beispiel 6

9 Teile Poly(butylmethacrylat-co-(2-dimethylaminoethyl)methacrylat-co-methylmethacrylat) (Eudragit^{®} E 100) werden in 60 Teilen Isopropanol und 4 Teilen Wasser gelöst. In diese Lösung werden 27 Teile mikronisiertes Vardenafil Hydrochlorid Trihydrat suspendiert. Die Suspension wird zur Trockene eingedampft, der Rückstand wird entnommen, gemahlen und gesiebt. 4,16 g des so erhaltenen Copräcipitates werden mit 5 g einer Farbvormischung bestehend aus 95 Teilen Pharmaburst^{®} B2, 4,4 Teilen gelbem Eisenoxid und 0,6 Teilen rotem Eisenoxid" 0,5 g pulverförmigem Orangenaroma, 1 g Aspartam, 86,34 g Pharmaburst^{®} B2 und 3 g Magnesiumstearat gemischt und zu Tabletten von 11 mm Durchmesser und einer Masse von 380 mg verpresst. Die so erhaltenen, schnell im Mund zerfallenden Tabletten setzen in der USP Blattrührerapparatur in 900 ml physiologischer Kochsalzlösung bei 37°C und 50 Umdrehungen pro Minute 47 % des Wirkstoffes in 5 Minuten frei.

### Beispiel 7

11,85 g Vardenafil Hydrochlorid Trihydrat mit einer mittleren Partikelgröße von 135 µm wird mit 1 g Magnesiumstearat 30 Minuten lang gemischt, durch ein 0,8 mm Sieb gesiebt und nochmals 30 Minuten lang gemischt. Nachfolgend werden 5 g Farbvormischung bestehend aus 95 Teilen Pharmaburst^{®} B2, 4,4 Teilen gelbem Eisenoxid und 0,6 Teilen rotem Eisenoxid, 0,5 g pulverförmiges Orangenaroma, 1 g Aspartam, 77,65 g Pharmaburst^{®} B2 und 3 g Magnesiumstearat zugesetzt und gemischt. Die pressfertige Mischung wird auf einer Tablettenmaschine zu runden Tabletten von 7 mm Durchmesser und einer Masse von 100 mg verpresst. Die so erhaltenen, schnell im Mund zerfallenden Tabletten setzen in der USP Blattrührerapparatur in 900 ml physiologischer Kochsalzlösung bei 37 °C und 50 Umdrehungen pro Minute 49 % des Wirkstoffes in 5 Minuten frei.

### Beispiel 8

2,44 g mikronisiertes Vardenafil Dihydrat, 0,68 g pulverisierte und durch 0,35 mm gesiebte Weinsäure und 45,37 g Pharmaburst^{®} B2 werden 5 Minuten lang gemischt, durch ein 0,5 mm Sieb gesiebt und nochmals 5 Minuten lang gemischt. Es wird 1,5 g Magnesiumstearat zugegeben und nochmals 5 Minuten lang gemischt. Diese Mischung wird auf einer Tablettenmaschine zu runden Tabletten von 9 mm Durchmesser und einer Masse von 220 mg verpresst. Die so erhaltenen, schnell im Mund zerfallenden Tabletten setzen in der USP Blattrührerapparatur in 900 ml physiologischer Kochsalzlösung bei 37°C und 50 Umdrehungen pro Minute 45 % des Wirkstoffes in 5 Minuten frei.

### Beispiel 9

10,0 g wasserfreie Vardenafil Base, 7,6 g Crospovidone, 38 g Calciumsilikat, 57 g mikrokristalline Cellulose, 246,5 sprühgetrocknetes Mannit, 3,8 g Aspartam und 1,9 g pulverförmiges Orangenaroma werden gemischt und trocken granuliert. Das Granulat wird mit 3,8 g hochdispersem Siliciumdioxid und 11,4 g Magnesiumstearat nachgemischt und zu Tabletten von 11 mm Durchmesser und einer Masse von 380 mg verpresst.

### Beispiel 10

130 g wasserfreies Vardenafil wird mit 24.7 g Orangenaroma, 49,4 g Aspartam und 4563 g Pharmaburst^{®} B2 (handelsübliche Hilfsstoffmischung der Firma SPI gemischt, 0.5 mm gesiebt, nochmals gemischt und auf einer Walze trocken granuliert. Dem Granulat werden 24,7 g hochdisperses Siliziumdioxid und 148,2 g Magnesiumstearat zugesetzt und in einem Freifallmischer 5 Minuten lang gemischt. Die Mischung wird auf einer Tablettenmaschine zu Tabletten mit einer Masse von 380 mg verpresst. Die schnell im Mund zerfallenden Tabletten erfüllen das erfindungsgemäße Lösegeschwindigkeitskriterium, da sich in 900 ml physiologischer Kochsalzlösung bei 37°C innerhalb von 5 Minuten nur etwa 26 % der applizierten Dosis freigesetzt werden.

### Beispiel 11

107 g wasserfreies Vardenafil und 536 g Erythritol werden in 10,7 kg Ethanol 80 % gelöst und in einer Vakuumwirbelschichtapparatur auf 1,5 kg Pharmaburst^{®} B2 aufgesprüht. Die so hergestellte feste Lösung von Vardenafil wird mit 20,4 g pulverförmigem Orangenaroma, 40,71 g Aspartam, 1745,4 g Pharmaburst^{®} B2 und 122,1 g Magnesiumstearat nachgemischt und auf einer Rundlauftablettenpresse mit plasmaverchromten Stempeln zu Tabletten von 11 mm Durchmesser und einer Masse von 380 mg verpresst.

## Patentansprüche

1. Arzneimittelformulierung enthaltend Vardenafil, die im Mund schnell zerfällt und über eine kontrollierte Bioverfügbarkeit verfügt, **dadurch gekennzeichnet, dass** die Wirkstofffreisetzung nach 5 Minuten in der USP Blattrührerapparatur bei 50 Umdrehungen pro Minute in physiologischer Kochsalzlösung bei 37°C weniger als 50 % der enthaltenen Dosis an Vardenafil beträgt.

2. Arzneimittelformulierung gemäß Anspruch 1, enthaltend Vardenafil Dihydrat oder wasserfreie Vardenafil Base.

3. Arzneimittelformulierung gemäß Anspruch 1, enthaltend Vardenafil Hydrochlorid Trihydrat mit einer mittleren Partikelgröße größer 80 µm.

4. Arzneimittelformulierung, gemäß Anspruch 1 enthaltend ein Vardenafil Salz mit einer physiologisch verträglichen Säure oder eine Mischung aus Vardenafil und einer physiologisch verträglichen Säure **dadurch gekennzeichnet, dass** der Wirkstoff und/oder die Säure von einem Polymer umhüllt oder in eine Polymermatrix eingebettet sind.

5. Arzneimittelformulierung gemäß Anspruch 4 enthaltend ein Vardenafil Salz mit einer physiologisch verträglichen Säure oder eine Mischung aus Vardenafil und einer physiologisch verträglichen Säure **dadurch gekennzeichnet, dass** der Wirkstoff und/oder die Säure von einem Polymer umhüllt oder in eine Polymermatrix eingebettet sind, wobei das Polymer in Speichel unlöslich und in Magensaft löslich ist.

6. Arzneimittelformulierung gemäß Anspruch 5, wobei das Polymer Poly(butylmethacrylat-co-(2-dimethylaminoethyl)methacrylat-co-methylmethacrylat) (Eudragit^{®} E 100 oder Eudragit^{®} E PO) darstellt.

7. Arzneimittelformulierung gemäß Anspruch 4, wobei das Polymer Ethylcellulose darstellt.
